# EUROPEAN PATENT APPLICATION

(11) **EP 4 197 519 A1**
(43) Date of publication of application: **21.06.2023**
(21) Application number: 22213516.2
(22) Date of filing: 14.12.2022
(51) Int. Cl.: A61K 8/02, A61K 8/37, A61K 8/92, A61K 8/9789, A61Q 17/00, A61Q 19/00

(54) **PRESERVATIVE COMPOSITIONS AND METHODS OF USE**

(30) Priority: 15.12.2021 US 202163290000 P
(71) Applicant: DPP Technologies Inc., Totonto ON M6P 1Z2 (CA)
(72) Inventor: Tawashi, Mona, Toronto, M6P 1Z2 (CA); Tawashi, Rashad, Toronto, M6P 1Z2 (CA)
(74) Representative: Studio Torta S.p.A.

(57) **Abstract**

Methods are provided for reducing or preventing undesirable changes in a composition over time, such as microbial growth, oxidative damage, and/or color changes. Date palm pollen or an extract thereof serves as a preservative, antimicrobial, and/or antioxidant component, thereby stabilizing the composition.

## Description

### CROSS-REFERENCE TO RELATED APPLICATIONS

This application claims the benefit of U.S. Provisional Application No. 63/290,000, filed on December 15, 2021, which is incorporated herein by reference in its entirety.

### FIELD OF THE INVENTION

The field relates to palm pollen or an extract thereof, and formulations thereof, for use in preservative and/or antimicrobial applications of use.

### BACKGROUND

The pharmaceutical, cosmetic, and food industries are highly concerned with the shelf life, quality, and integrity of their products. This concern is also shared with regulatory agencies that regulate these products and with consumers who purchase them. Research is ongoing to identify ideal preservatives that can prolong the shelf life of products within the standards of particular industries, and this represents a very important economic concern and challenge.

There is a need for a preservative that is natural and broad spectrum. The preservative should act within the formulation as an integral part of the product. The preservative should be able to withstand manufacturing processes, such as grinding, homogenization, and exposure to heat. It also should increase the stability of the active materials in the formulation, prevent degradation of active materials or excipients used in the formulation, and maintain integrity of the individual chemical constituents and the product as a whole.

### BRIEF SUMMARY OF THE INVENTION

In one aspect, a composition is provided that includes pollen from a palm species of the genus *Phoenix* or an extract thereof, wherein the composition is resistant to microbial growth and/or oxidative damage or color change (*e.g*., photodegradation) over a period of time of at least about 3 years at about 20 °C. In some embodiments, the composition is resistant to microbial growth and/or oxidative damage and/or color change (*e.g*., photodegradation) for a time period of any of at least about 1, 2, 3, 4, 5, 6, 7, 8, 9, 10, or 11 months, or any of at least about 1, 1.5, 2, 2.5, 3, 3.5, 4, 4.5, 5, 5.5, 6, 6.5, 7, 7.5, 8, 8.5, 9, 9.5, or 10 years, or longer. Microbial growth and/or oxidative damage is prevented or is reduced in the composition in comparison to an identical or substantially identical composition that does not include the pollen or extract thereof. The pollen or extract thereof acts as a stabilizer and/or as a preservative in the composition. In some embodiments, the pollen or extract thereof is from *Phoenix dactylifera* L.

In some embodiments, the composition is anhydrous.

In some embodiments, the pollen or extract thereof is included in the composition at a concentration of about 2% (w/w) to about 20% (w/w).

In some embodiments, the composition does not substantially degrade over the period of time, as indicated by a reduction or elimination of changes in consistency, color, and/or smell, and/or reduction or elimination of microbial growth, in comparison to an identical or substantially identical composition that does not include the pollen or extract thereof.

In some embodiments, bacterial growth that is limited in the composition to a maximum of 100 CFU per g or ml. In some embodiments, fungal growth is limited in the composition to a maximum of 10 CFU per g or ml over said period of time. In some embodiments, bacterial and/or fungal growth is limited in the composition to a maximum of 10⁶ CFU per g or ml.

In some embodiments, the composition is stored in a transparent or translucent container, e.g., and is not shielded from exposure to light, for the period of time and does not degrade due to photodegradation.

In some embodiments, the composition is formulated as a skin care, cosmetic, food, or pharmaceutical composition. In some embodiments, the composition is a liquid, suspension, emulsion, or powder composition, such as an anhydrous liquid, suspension, emulsion, or powder composition. In some embodiments, the composition includes a lipid.

In some embodiments, the composition includes one or more constituent to facilitate formulation, stability, and/or topical application of the composition. For example, the one or more constituent may include, but is not limited to, a flow regulating agent, a filler, an excipient, an alcohol, a suspending agent, an oil phase, a humectant, and/or a thickener.

In some embodiments, the composition may include one or more component to facilitate topical application of the composition. For example, the topical composition may be in the form of a powder, a compact disc, a suspension, a cream, a lotion, an ointment, a gel, a foam, a paste, an aerosol, a body wash, a hair product, a sunscreen, a lipstick, an emulsion, a cosmetic, or an anhydrous absorption base composition. For example, the one or more constituent selected may include, but is not limited to, colloidal silica, titanium dioxide, isopropyl alcohol, benzalkonium chloride, stearic acid, cetyl alcohol, isopropyl palmitate, methylparaben, propylparaben monostearate, sorbitol, polysorbate, milk, coconut oil, almond oil, lanolin, lecithin, and/or beeswax.

In some embodiments, a composition as described herein may include a fragrance.

In some embodiments, the composition may include one or more component to facilitate oral administration of the composition. For example, the oral composition may be in the form of a powder, a tablet, a capsule, an aerosol suspension, or an effervescent powder or tablet.

In some embodiments, the composition is formulated as a food composition. For example, the food composition may be in the form of an oil, a dehydrated food, or a powdered food or baking mix.

In another aspect, a method is provided for preserving and/or stabilizing a composition. The method includes adding pollen or an extract thereof from a palm species of the genus *Phoenix* to a composition, and microbial growth and/or oxidative damage or color change (*e.g*., photodegradation) is reduced or prevented over a period of time of at least about 3 years at about 20 °C, in comparison to an identical or substantially identical composition that does not include the pollen or extract thereof. In some embodiments, the composition is resistant to microbial growth and/or oxidative damage and/or color change (e.g., photodegradation) for a time period of any of at least about 1, 2, 3, 4, 5, 6, 7, 8, 9, 10, or 11 months, or any of at least about 1, 1.5, 2, 2.5, 3, 3.5, 4, 4.5, 5, 5.5, 6, 6.5, 7, 7.5, 8, 8.5, 9, 9.5, or 10 years, or longer. In some embodiments, the pollen or extract thereof is from *Phoenix dactylifera* L.

In some embodiments, the composition is anhydrous.

In some embodiments, the pollen or extract thereof is added to the composition at a concentration of about 2% (w/w) to about 10% (w/w).

In some embodiments, the composition is stored in a transparent or translucent container for the period of time. In some embodiments, the composition is not shielded from exposure to light, and does not degrade due to photodegradation.

In another aspect, a preservative composition is provided, which includes pollen from a palm species of the genus *Phoenix* (for example, *Phoenix dactylifera* L.) or an extract thereof, and at least one diluent. The preservative composition reduces or eliminates microbial growth and/or oxidative damage and/or color change (*e.g*., photodegradation) in a product or composition into which it is incorporated, in comparison to an identical or substantially identical product or composition that does not include the preservative composition, e.g., does not include the pollen or extract thereof. In some embodiments, the preservative composition is a liquid preparation, and the diluent is a liquid such as a hydrophobic oil. In other embodiments, the preservative composition is a dry preparation, and the diluent is a powder, such as titanium dioxide. In some embodiments, the preservative composition is anhydrous.

In some embodiments, when the preservative composition is incorporated into a drug or cosmetic composition, bacterial growth is limited to a maximum of 100 CFU per g or ml and/or fungal growth is limited to a maximum of 10 CFU per g or ml, over a period of time of at least about 3 years (or a time period of any of at least about 1, 2, 3, 4, 5, 6, 7, 8, 9, 10, or 11 months, or any of at least about 1, 1.5, 2, 2.5, 3, 3.5, 4, 4.5, 5, 5.5, 6, 6.5, 7, 7.5, 8, 8.5, 9, 9.5, or 10 years, or longer) at about 20 °C.

In some embodiments, when the preservative composition is incorporated into a food composition, bacterial and/or fungal growth is limited to a maximum of 10⁶ CFU per g or ml, over a period of time of at least about 3 years (or a time period of any of at least about 1, 2, 3, 4, 5, 6, 7, 8, 9, 10, or 11 months, or any of at least about 1, 1.5, 2, 2.5, 3, 3.5, 4, 4.5, 5, 5.5, 6, 6.5, 7, 7.5, 8, 8.5, 9, 9.5, or 10 years, or longer) at about 20 °C.

In some embodiments, the preservative composition is incorporated into a composition or product, such as a drug, cosmetic, or food product. at a concentration of about 2% (w/w) to about 20% (w/w).

In some embodiments, the composition or product into which the preservative composition is incorporated includes at least one lipid.

In another aspect, a composition or product, such as a drug, cosmetic, or food product, which includes a preservative composition as described herein (*e.g.,* a preservative composition that includes pollen from a palm species of the genus *Phoenix* (for example, *Phoenix dactylifera* L.) or an extract thereof, and at least one diluent, is provided. The composition or product resists or contains less microbial growth and/or oxidative damage and/or photodegradation, in comparison to an identical or substantially identical product or composition that does not include the preservative composition, *e.g*., does not include the pollen or extract thereof, for example, for a time period of any of at least about 1, 2, 3, 4, 5, 6, 7, 8, 9, 10, or 11 months, or any of at least about 1, 1.5, 2, 2.5, 3, 3.5, 4, 4.5, 5, 5.5, 6, 6.5, 7, 7.5, 8, 8.5, 9, 9.5, or 10 years, or longer).

### DETAILED DESCRIPTION

Provided herein are compositions that include palm pollen in an amount that is effective to serve as a preservative, stabilizer, antimicrobial substance, and/or antioxidant. Inclusion of palm pollen in the composition may reduce or prevent microbial growth and/or may reduce or prevent color change and/or oxidative damage to the composition or to components of the composition, in comparison to an identical composition that does not contain the palm pollen. DPP may serve as a natural preservative, stabilizer, antimicrobial, and/or antioxidant.

Unless defined otherwise herein, all technical and scientific terms used herein have the same meaning as commonly understood by one of ordinary skill in the art to which this invention belongs. Singleton, et al., Dictionary of Microbiology and Molecular Biology, second ed., John Wiley and Sons, New York (1994), and Hale & Markham, The Harper Collins Dictionary of Biology, Harper Perennial, NY (1991) provide one of skill with a general dictionary of many of the terms used in this invention. Any methods and materials similar or equivalent to those described herein can be used in the practice or testing of the present invention.

Numeric ranges provided herein are inclusive of the numbers defining the range.

### Definitions

"A," "an" and "the" include plural references unless the context clearly dictates otherwise.

The term "about" is used herein to mean plus or minus ten percent (10%) of a value. For example, "about 100" refers to any number between 90 and 110.

The terms "and/or" and "or" are used interchangeably herein and refer to a specific disclosure of each of the two specified features of components with or without the other. Thus, the term "and/or" as used in a phrase such as "A and/or B" herein is intended to include "A and B," "A or B," "A" (alone), and "B" (alone). Likewise, the term "and/or: as used in a phrase such as "A, B and/or C" is intended to encompass each of the following aspects: A, B and C; A, B or C; A or C; A or B; B or C; A and C; A and B; B and C; A (alone); B (alone); and C (alone.

The term "antimicrobial" refers to any agent or combination of agents which is intended to kill, inactivate or inhibit the growth of any microbes such as bacteria, fungi, viruses, yeast, mold, and the like. The terms "antimicrobial" and "biocidal" are used interchangeably herein.

The term "broad spectrum" refers to an antimicrobial substance that acts against a wide range of microorganisms, for example, Gram-positive bacteria, Gram-negative bacteria, fungi, etc.

The terms "DPP," "date palm pollen," or "palm pollen," used interchangeably herein, refer to pollen from a species of the genus *Phoenix* in the family *Palmae.* The term "date palm" as used herein includes both ornamental and fruit-producing species in the genus *Phoenix.* As used herein, the term an "extract" of DPP refers to material that has been extracted from pollen from a species of the genus *Phoenix,* or one or more compound(s) that is derived or extracted from DPP, such as, but not limited to, flavonoid(s), quercetin, triterpene(s), and/or other constituent compound(s) of DPP.

The term "derived from" encompasses the terms "originated from," "obtained from," "obtainable from," "isolated from," "extracted from," and "created from," and generally indicates that one specified material finds its origin in another specified material or has features that can be described with reference to another specified material.

"Effective amount" as used herein refers to an amount (*e.g*., minimum inhibitory concentration (MIC)) of a preservative composition as disclosed herein that is sufficient to prevent or inhibit microbial growth. The preservative compositions described herein may be active against Gram-positive bacteria, Gram-negative bacteria, yeast, fungi, and/or molds.

An "individual" refers to a mammal, often a human.

The terms "microorganism" and "microbe" are used interchangeably herein and refer to microscopic organisms, *e.g*., microscopic single celled life forms, including but not limited to bacteria, fungi, and algae microorganisms. Microbes may exist in single-celled form or in a colony of cells or in a biofilm. Microbes include eukaryotes and prokaryotes. Nonlimiting examples of microbes include bacteria, fungi, algae, archaea, protozoa, viruses, and the like.

The term "pharmaceutical" refers to a substance, *e.g*., a chemical substance, which may be used in medical treatment, cure, or prevention of a condition for which administration of the substance is beneficial, such as a disease condition.

The term "preservative" refers to a substance or agent that is added to a product to prevent decomposition by microbial growth and/or by undesirable chemical changes.

The terms "recovered," "isolated," "purified," "extracted," and "separated" as used herein refer to a material (*e.g*., a protein, nucleic acid, cell, or small molecule) that is removed from at least one component with which it is naturally associated. For example, these terms may refer to a material which is substantially or essentially free from one or more components which normally accompany it as found in its native state, such as, for example, an intact biological system, such as, for example, intact DPP.

The term "shelf life" refers to the length of time for which an item (*e.g*., a product as described herein) remains usable, fit for consumption, or saleable, *e.g*., free from microbial contamination, color changes, etc.

The term "topical formulation" as used herein refers to a composition that is formulated for application to an external body surface, such as skin or mucous membranes.

"Unit dose" refers to an amount of a substance contained in a formulation for topical administration as described herein that is sufficient to cause a therapeutic, prophylactic, or cosmetic effect when applied to an external body surface of an individual. A unit dose may be administered in a single topical application of the formulation or in two or more applications.

### Date palm pollen

The compositions and methods described herein include pollen from a palm in the genus *Phoenix* and/or an extract thereof. In some embodiments, pollen from a date palm is used. In one exemplary embodiment, the formulations herein include pollen from the species *Phoenix dactylifera L, Palmae.* The pollen can be harvested from male date palm trees during the flowering season in the months of February and March.

Pollen grains have been designed by nature to transfer genetic material from plant-to-plant. Date palm pollen (DPP) exists in a very fine powder material, produced by the male flowering date palm. The male flower develops 2-3 weeks before the female flower. Once the male pods open, they may be removed from the tree and dried. Once dried, pollen may be stored in a cool environment. DPP represents the reproductive cell of the male flower and contains the male contribution to the next generation of the plant.

### Physico-chemical Characteristics

DPP has unique size, shape and surface characteristics. Physico-chemical interaction of DPP with a biological surface may depend, for example, on particle size, micro-morphology, and/or surface geometry. Properties like flow, adhesion, and biochemical interactions with body surfaces may be under the influence of any or all of these characteristics.

The size of DPP pollen grains is between 12 to 30 microns, for example, about 12, 15, 20, 25 or 30 microns with narrow particle size distribution. In one embodiment, DPP has an average diameter of about 24 microns using microscopic analysis. For example, in one embodiment, DPP is from the species *Phoenix dactylifera L, Palmae* and has a diameter of about 21 to about 27 microns, *e.g*., about 21, 22, 23, 24, 25, 26, or 27 microns, for example, a mean diameter of about 24 microns.

The shape is frequently elliptical. The surface is reticulate with irregular semicircular pores and covered with spikes or needle like structures. The tip edges of the spikes are in the submicron range.

Dry DPP powder has excellent flow properties in addition to excellent spreading and adhesion properties on surfaces. Adhesion on surfaces takes place by mechanical interlocking. DPP has both adhesive and spreading properties when applied to body surfaces. The flow properties of freshly-collected pollen depend on its moisture content; when dry, it is free flowing. The flow properties can be regulated by the addition of one or more flow regulating agent(s). Surface ruggedness of palm pollen permits it to adhere easily to biological surfaces. The spikes present on the surface allow the pollen to adhere to complex surface geometry and reach difficult-to-reach cracks on the skin surface.

### Phytochemical Characteristics

Early investigations revealed that DPP contains estrone. (Hassan and Abou el Wafa (1947) Nature 159(4038): 409) More recent studies have confirmed that all three major naturally-occurring estrogens (estrone, estradiol, and estriol) are present in DPP. (Fawkeya and Ateya (2011) Australian Journal of Basic and Applied Science 5(8): 606-614) DPP also contains rutin, a glycoside combination of the flavonol quercetin and the disaccharide rutinose that acts as an antioxidant. DPP also contains carotenoids, which are efficient freeradical scavengers, known to enhance the vertebrate immune system. A glucoprotein with gonadotrophic activity has been isolated from DPP. (Mahran et al. (1976) Planta Med. 29(2):171-175; El Ridi et al. (1952) Arch Biochem Biophys 39(2):317-21) DPP also contains triterpenoids (*e.g.,* beta-amyrin), natural lipids (*e.g.,* esters of palmitic, linoleic, and myristic acid), minerals (*e.g*., magnesium, phosphorous, sulfur, potassium, calcium, zinc, and/or manganese), vitamins (*e.g*., vitamin B₁, B₂, and/or B₁₂), and/or amino acids (*e.g*., essential amino acids, *e.g*., threonine, valine, methionine, isoleucine, leucine, phenylalanine, histidine, and/or lysine) and/or non-essential amino acids (*e.g*., aspartic acid, glutamic acid, proline, glycine, alanine, tyrosine, arginine, and/or serine). (Sebii, et al. (2019) Advances in Food Technology and Nutritional Sciences 5(3):84-91; Abdel-Shaheed, et al. (2021) Food and Nutrition Sciences 12:147-161)

### Preservative properties

As described herein, DPP or an extract thereof may be provided in a composition as a natural preservative substance, alone or in combination with one or more other preservative substance(s), to prevent microbial growth in the composition, and/or oxidation, color change, and/or other degradative effects, including, but not limited to, photodegradation, of one or more components of the composition.

Although not wishing to be bound by theory, bioactive constituents embedded within the pollen grains may act as preservative, antibacterial, antifungal, stabilizer, and/or antioxidant agents, when included in a composition as described herein.

Triterpene and/or other constituents of DPP may provide antimicrobial (*e.g*., antibacterial and/or antifungal) effects, *e.g*., prevent or reduce bacterial and/or fungal growth in the composition.

DPP may also prevent color fading, *e.g*., changes in color due to photo-degradation and/or oxidative stress, maintaining product appearance and integrity, *e.g*., even when stored in a translucent or transparent container. Flavonoid and/or other components of DPP may act as antioxidants, preventing or reducing oxidative stress. Quercetin and/or other components of DPP may block UV radiation ( UVA, UVB) which would otherwise be responsible for breaking of chemical bonds in coloring agents. The antioxidant (*e.g*., flavonoid) and UV blocking (*e.g*., quercetin) components may work together additively or synergistically to reduce or prevent degradation of coloring agents or compounds, such as, but not limited to FD&C colors used in products such as food, drug, and cosmetic products.

### Methods

Methods are provided herein for preserving and/or stabilizing a composition. The methods include adding or including DPP or an extract thereof from a palm species of the genus *Phoenix* to a composition. The pollen or extract thereof provides a preservative and/or stabilizing effect, such as prevention or reduction of microbial (*e.g*., bacterial and/or fungal) growth, oxidative damage, photodegradation, color change, odor, and/or optical properties such as cloudiness, to the composition for a time period of at least about 1, 2, 3, 4, 5, 6, 7, 8, 9, 10, or 11 months, or at least about 1, 1.5, 2, 2.5, 3, 3.5, 4, 4.5, 5, 5.5, 6, 6.5, 7, 7.5, 8, 8.5, 9, 9.5, or 10 years, or longer, in comparison to an identical or substantially identical composition that does not include the DPP or extract thereof. In some embodiments, the composition is stored at an average temperature of about 20 °C (*e.g*., ambient temperature) for the time period. In some embodiments, the composition is anhydrous or substantially anhydrous. In other embodiments, the composition is not anhydrous. In some embodiments, the composition is stored in a transparent or translucent container and the container is not shielded or blocked from exposure to light. In one embodiments, the DPP or extract thereof is from *Phoenix dactylifera L.*

In some embodiments, the DPP or extract thereof is included in the composition at a concentration of any of at least about 2% (w/w), 3% (w/w), 4% (w/w), 5% (w/w), 6% (w/w), 7% (w/w), 8% (w/w), 9% (w/w), 10% (w/w), 11% (w/w), 12% (w/w), 13% (w/w), 14% (w/w), 15% (w/w), 16% (w/w), 17% (w/w), 18% (w/w), 19% (w/w), or 20% (w/w), or any of about 2% (w/w) to about 20% (w/w), 2% (w/w) to about 10% (w/w), about 2% (w/w) to about 4% (w/w), about 3% (w/w) to about 5% (w/w), about 4% (w/w) to about 6% (w/w), about 5% (w/w) to about 7% (w/w), about 6% (w/w) to about 8% (w/w), about 8% (w/w) to about 10% (w/w), about 2% (w/w) to about 5% (w/w), about 3% (w/w) to about 6% (w/w), about 4% (w/w) to about 7% (w/w), about 5%(w/w) to about 8% (w/w), about 6% (w/w) to about 9% (w/w), about 7% (w/w) to about 10% (w/w), about 2% (w/w) to about 6% (w/w), about 3% (w/w) to about 7% (w/w), about 4% (w/w) to about 8% (w/w), about 5% (w/w) to about 9% (w/w), about 6% (w/w) to about 10% (w/w), about 2% (w/w) to about 7% (w/w), about 2% (w/w) to about 8% (w/w), about 4% (w/w) to about 10% (w/w), about 5% (w/w) to about 10% (w/w), about 10% (w/w) to about 15% (w/w), about 15% (w/w) to about 20% (w/w), about 8% (w/w) to about 15% (w/w), about 5% (w/w) to about 12% (w/w), or about 8% (w/w) to about 20% (w/w).

In some embodiments, the DPP or extract thereof reduces or prevents microbial growth in the composition. For example, bacterial and/or fungal (*e.g*., yeast and/or mold) growth may be limited in the composition to a maximum of 10 CFU, 100 CFU, 1000 CFU, 10⁴ CFU, 10⁵ CFU, or 10⁶ CFU per g or ml.

In some embodiments, the composition that includes DPP or an extract thereof does not degrade or substantially does not degrade over the period of time, as indicated by a reduction or elimination of changes in consistency, viscosity, color, optical properties such as cloudiness, and/or odor over the period of time, in comparison to an identical or substantially identical composition that does not include the DPP or extract.

### Compositions

Compositions are provided herein in which DPP or an extract thereof, or one or more compound(s) that are derived or extracted from DPP, such as, but not limited to, flavonoid(s), quercetin, triterpene(s), and/or other constituent compound(s) of DPP, is included and serves as a preservative and/or stabilizer, and/or reduces or prevents microbial growth, thereby increasing shelf life of the composition. For example, DPP or an extract thereof may be included in a composition, such as, but not limited to, a cosmetic, skin care, pharmaceutical, or food composition. As an example, spoilage of a food product (*e.g*., microbial growth in a food product) may be prevented or significantly reduced by inclusion of DPP or an extract thereof, thereby increasing shelf life.

In one embodiments, the DPP or extract thereof is from *Phoenix dactylifera L.*

DPP or an extract thereof is included at a concentration that is effective to prevent degradation of the composition over time, such as microbial growth, color change, cloudiness, change in consistency (*e.g*., viscosity), and/or oxidation of the composition or one or more components of the composition. The DPP or extract may act as a preservative, stabilizer, and/or antioxidant in the composition. In some embodiments, an effective amount of the DPP or equivalent amount of DPP in an extract thereof, or one or more component(s) thereof, such as, but not limited to, flavonoid(s), quercetin, triterpene(s), and/or other constituent compound(s) of DPP or extracted from DPP, is any of at least about any of 2% (w/w), 3% (w/w), 4% (w/w), 5% (w/w), 6% (w/w), 7% (w/w), 8% (w/w), 9% (w/w), 10% (w/w), 11% (w/w), 12% (w/w), 13% (w/w), 14% (w/w), 15% (w/w), 16% (w/w), 17% (w/w), 18% (w/w), 19% (w/w), or 20% (w/w), or any of about 2% (w/w) to about 20% (w/w), or any of about 2% (w/w) to about 10% (w/w), about 2% (w/w) to about 4% (w/w), about 3% (w/w) to about 5% (w/w), about 4% (w/w) to about 6% (w/w), about 5% (w/w) to about 7% (w/w), about 6% (w/w) to about 8% (w/w), about 8% (w/w) to about 10% (w/w), about 2% (w/w) to about 5% (w/w), about 3% (w/w) to about 6% (w/w), about 4% (w/w) to about 7% (w/w), about 5%(w/w) to about 8% (w/w), about 6% (w/w) to about 9% (w/w), about 7% (w/w) to about 10% (w/w), about 2% (w/w) to about 6% (w/w), about 3% (w/w) to about 7% (w/w), about 4% (w/w) to about 8% (w/w), about 5% (w/w) to about 9% (w/w), about 6% (w/w) to about 10% (w/w), about 2% (w/w) to about 7% (w/w), about 2% (w/w) to about 8% (w/w), about 4% (w/w) to about 10% (w/w), about 5% (w/w) to about 10% (w/w)), about 10% (w/w) to about 15% (w/w), about 15% (w/w) to about 20% (w/w), about 8% (w/w) to about 15% (w/w), about 5% (w/w) to about 12% (w/w), or about 8% (w/w) to about 20% (w/w).

The composition with an effective amount of DPP or extract or constituent compound(s) thereof may resist microbial growth, oxidation, and/or color change, *e.g.,* microbial growth, oxidation, and/or color change is reduced or prevented in the composition, for a period of any of at least 1, 1.5, 2, 2.5, 3, 3.5, 4, 4.5, 5, 5.5, 6, 6.5, 7, 7.5, 8, 8.5, 9, 9.5, or 10 years, or longer, for example, when stored at a temperature of about 20 °C, *e.g.,* at an average temperature of about 20 °C, *e.g.,* at ambient temperature, in comparison to an identical composition that does not include the DPP.

In some embodiments, the DPP or extract thereof or constituent compound(s) thereof, has an antimicrobial effect in the composition. For example, microbial growth may be enumerated as described in USP 61 (<61> Microbiological Examination of Nonsterile Products: Microbial Enumeration Tests, Sixth Interim Revision Announcement, November 21, 2016). In some embodiments, bacterial and/or fungal (*e.g*., yeast and/or mold) growth is limited in the composition to a maximum of 10 CFU, 100 CFU, 1000 CFU, 10⁴ CFU, 10⁵ CFU, or 10⁶ CFU per g or ml. In some embodiments, the composition is a drug or cosmetic composition, or a topical composition, and bacterial growth is limited to a maximum of 100 CFU per g or ml, and/or fungal growth is limited to 10 CFU per g or ml. In some embodiments, the composition is a food composition, and bacterial and/or fungal growth is limited to a maximum of 10⁶ CFU per g or ml.

In certain embodiments, the composition is anhydrous. In other embodiments, the composition is not anhydrous. In some embodiments, the composition may include a lipid.

The composition may be a food composition, such as, but not limited to, a prepared food composition, a dehydrated food, or a powdered food, such as a baking mix. In one embodiment, the composition is an oil, such as a culinary or cooking oil. In some embodiments, the food composition is anhydrous or substantially anhydrous.

The composition may be a cosmetic or sunscreen composition, and may optionally include one or more constituent to facilitate topical application of the composition, such as, but not limited to, a flow regulating agent, a filler, an excipient, an alcohol, a suspending agent, an oil phase, a humectant, or a thickener. In some embodiments, the cosmetic or sunscreen composition is anhydrous or substantially anhydrous. In some embodiments, a composition for topical administration in the form of an aerosol or other type of spray formulation, or a suspension, such as a shampoo or hair product, may contain about 0.05 g to about 0.40 g, about 0.10 g to about 0.40 g, about 0.15 g to about 0.40 g, about 0.20 g to about 0.40 g, about 0.25 g to about 0.40 g, about 0.30 g to about 0.40 g, about 0.35 g to about 0.40 g, about 0.05 g to about 0.10 g, about 0.05 g to about 0.15 g, about 0.05 g to about 0.20 g, about 0.05 g to about 0.25 g, about 0.05 g to about 0.30 g, about 0.05 g to about 0.35 g, about 0.10 to about 0.15 g, about 0.15 g to about 0.20 g, about 0.20 g to about 0.25 g, about 0.25 g to about 0.30 g, about 0.30 g to about 0.35 g, about 0.35 g to about 0.40 g, about 0.10 g to about 0.25 g, about 0.15 g to about 0.30 g, about 0.20 g to about 0.35 g, about 25 g to about 0.40 g, about 0.10 g to about 0.30 g, or about 0.20 g to about 0.40 g of palm pollen, or any of about 0.05, 0.10, 0.15, 0.20, 0.25, 0.30, 0.35, or 0.40 g of palm pollen, or an equivalent amount of extract or constituent compound(s) thereof.

The composition may be a pharmaceutical or a composition formulated for oral administration, and may optionally be in the form of a powder, a tablet, a capsule, an aerosol suspension, or an effervescent powder or tablet. In some embodiments, the pharmaceutical or a composition formulated for oral administration is anhydrous or substantially anhydrous. In various embodiments, a composition for oral administration in the form of a powder, an effervescent powder, a tablet, an effervescent tablet, a capsule a suspension, a mouthwash, a toothpaste, or a lozenge may contain about 0.1 g to about 6.0 g, about 0.5 g to about 6.0 g, about 1.0 g to about 6.0 g, about 1.5 g to about 6.0 g, about 2.0 g to about 6.0 g, about 2.5 g to about 6.0 g, about 3.0 g to about 6.0 g, about 3.5 g to about 6.0 g, about 4.0 g to about 6.0 g, about 4.5 g to about 6.0 g, about 5.0 g to about 6.0 g, about 5.5 g to about 6.0 g, about 0.1 g to about 0.5 g, about 0.1 g to about 1.0 g, about 0.1 g to about 2.0 g, about 0.1 g to about 2.5 g, about 0.1 g to about 3.0 g, about 0.1 g to about 3.5 g, about 0.1 g to about 4.0 g, about 0.1 g to about 4.5 g, about 0.1 g to about 5.0 g, about 0.1 g to about 5.5 g, about 0.1 g to about .05 g, about 0.5 g to about 1.0 g, about 1.0 g to about 1.5 g, about 1.5 g to about 2.0 g, about 2.5 g to about 3.0 g, about 3.0 g to about 3.5 g, about 3.5 g to about 4.0 g, about 4.0 g to about 4.5 g, about 4.5 g to about 5.0 g, about 5.5 g to about 6.0 g, about 0.1 g to about 1.0 g, about 1.0 g to about 2.0 g, about 2.0 g to about 3.0 g, about 3.0 g to about 4.0 g, about 4.0 g to about 5.0 g, about 5.0 g to about 6.0 g, about 0.1 g to about 1.5 g, about 1.0 g to about 3.0 g, about 2.0 g to about 4.0 g, about 3.0 g to about 5.0 g, about 1.5 g to about 3.0 g, about 2.5 g to about 5.0 g, or about 3.5 g to about 6.0 g of palm pollen, or any of about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4,2.5,2.6,2.7,2.8,2.9,3.0,3.1,3.2,3.3,3.4,3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, or 6.0 g of palm pollen, or an equivalent amount of extract or constituent compound(s) thereof.

In some embodiments, a composition in the form of an aerosol or other type of spray formulation for oral administration may contain about 0.05 g to about 0.50 g, about 0.10 g to about 0.50 g, about 0.15 g to about 0.50 g, about 0.20 g to about 0.50 g, about 0.25 g to about 0.50 g, about 0.30 g to about 0.50 g, about 0.35 g to about 0.50 g, about 0.40 g to about 0.50 g, about 0.45 g to about 0.50 g, about 0.05 g to about 0.10 g, about 0.05 g to about 0.15 g, about 0.05 g to about 0.20 g, about 0.05 g to about 0.25 g, about 0.05 g to about 0.30 g, about 0.05 g to about 0.35 g, about 0.10 to about 0.15 g, about 0.15 g to about 0.20 g, about 0.20 g to about 0.25 g, about 0.25 g to about 0.30 g, about 0.30 g to about 0.35 g, about 0.35 g to about 0.40 g, about 0.40 to about 0.45, about 0.45 g to about 0.50 g, about 0.10 g to about 0.25 g, about 0.15 g to about 0.30 g, about 0.20 g to about 0.35 g, about 25 g to about 0.40 g, about 0.10 g to about 0.30 g, or about 0.20 g to about 0.40 g of palm pollen, about 0.30 g to about 0.50 g, or any of about 0.05, 0.10, 0.15, 0.20, 0.25, 0.30, 0.35, 0.40, 0.45, or 0.50 g of palm pollen, or an equivalent amount of extract or constituent compound(s) thereof.

In some embodiments of the formulations described herein, an extract of palm pollen is used. An extract may be prepared, for example, by extraction of one or more constituent(s), such as, but not limited to, flavonoid(s), quercetin, triterpene(s), and/or other constituent compound(s) of DPP, or all or substantially all constituents, of the pollen with one or more organic solvent(s). Extraction may be followed by evaporation, purification, and/or drying of the extract prior to incorporation into a formulation. A DPP extract may contain one or more components of DPP and no or substantially no intact pollen grains. In some embodiments, a formulation as described herein may contain both DPP and an extract of DPP.

Typically, in addition to the palm pollen or extract thereof, a composition herein includes one or more additional constituents to facilitate administration, such as flow regulator(s), diluent(s), filler(s), anti-adhesive(s), and/or compound(s) that produce an effervescent reaction. In some embodiments, one or more flow regulating agent(s) is included. For example, silica (e.g., colloidal silica), silica gel, magnesium trisilicate, cornstarch, and/or talc may be included as a flow regulating agent. A flow regulating agent may be included to increase flow of the formulation, depending on the application of use. A powder, tablet, or capsule formulation may include one or more such flow regulating agent(s). A tablet or capsule formulation may further contain one or more diluent(s), such as spray dried lactose and/or starch, and/or one or more anti-adhesive substance(s), such as magnesium stearate, talc, and/or stearic acid. Effervescent formulations, such as effervescent powders or tablets, may further contain compounds to produce an effervescent reaction, for example, when dissolved in water, *e.g*., acid and base components such as tartaric acid, citric acid, and/or sodium bicarbonate. An aerosol formulation may contain one or more propellant(s) to facilitate pressurized packing of the composition, such as dichlorodifluoromethane (Propellant 12). In some embodiments, one or more flavoring or fragrance substance may be included as suitable for the application of use (*e.g*., flavoring for oral administration or fragrance for topical administration).

In some embodiments, the composition is formulated as a powder, and in addition to palm pollen or an extract thereof, the powder formulation may contain one or flow regulator(s), such as silica (*e.g*., colloidal silica), silica gel, magnesium trisilicate, cornstarch, and/or talc. The powder formulation may be suspended in a liquid, such as water, prior to administration, *e.g.,* oral administration.

In some embodiments, the composition is formulated as an effervescent powder, and in addition to palm pollen or an extract thereof, the effervescent powder may contain one or more diluent(s), such as spray dried lactose and/or starch, and compound(s) that will produce an effervescent reaction when the powder is dissolved in a suitable liquid for the effervescent reaction to occur, such as water or other aqueous media (*e.g*., juice, milk, etc.). A nonlimiting example of compounds that will produce such an effervescent reaction is tartaric acid, citric acid, and sodium bicarbonate. An effervescent powder formulation may optionally include one or more flavoring substance (*e.g*., mint, fruit flavor, chocolate, etc).

In some embodiments, the composition is formulated as a tablet, and in addition to palm pollen or an extract thereof, the tablet may contain one or more diluent(s), such as spray dried lactose and/or starch, and one or more anti-adhesive(s), such as magnesium stearate, talc, and/or stearic acid.

In some embodiments, the composition is formulated as an effervescent tablet, and in addition to palm pollen or an extract thereof, the tablet may contain one or more diluent(s), such as spray dried lactose and/or starch, one or more anti-adhesive(s), such as magnesium stearate, talc, and/or stearic acid, and one or more flow regulator(s), such as silica (*e.g*., colloidal silica), silica gel, magnesium trisilicate, cornstarch, and/or talc, and compound(s) that will produce an effervescent reaction when the tablet is dissolved in a suitable liquid for the effervescent reaction to occur, such as water or other aqueous media (*e.g*., juice, milk, etc.). A nonlimiting example of compounds that will produce such an effervescent reaction is tartaric acid, citric acid, and sodium bicarbonate. An effervescent tablet formulation may optionally include one or more flavoring substance (*e.g*., mint, fruit flavor, chocolate, etc).

In some embodiments, the composition is formulated in a capsule (*e.g*., hard gelatin capsule), and in addition to palm pollen or an extract thereof, the tablet may contain one or more diluent(s), such as spray dried lactose and/or starch, and one or more flow regulator(s), such as silica (*e.g*., colloidal silica), silica gel, magnesium trisilicate, cornstarch, and/or talc.

In some embodiments, the composition is formulated as an aerosol, and in addition to palm pollen or an extract thereof, the aerosol may contain one or more propellant(s), such as dichlorodifluoromethane (Propellant 12).

In some embodiments, the composition is formulated as a suspension for topical administration, and in addition to palm pollen or an extract thereof, the suspension may contain a vehicle or carrier (*e.g*., suspending media) of appropriate viscosity and having the ability to suspend palm pollen, such as polyethylene glycol (*e.g.,* PEG 400). A topical suspension may optionally include one or more fragrance.

### EXAMPLES

The following examples are intended to illustrate, but not limit, the invention.

### Example 1

The following formulations were prepared:
A. 10% w/w DPP serum formulation: almond oil, 200 ml; DPP, 20 g; colloidal silica, 3 g; fragrance, 2 ml or QS
B. 16% w/w DPP face cream: almond oil, 50 g; DPP, 20 g; isopropyl alcohol, 3 g; Carbowax^{™} 6000, 3 g; cocoa butter, 15 g; colloidal silica, 1 g; Span^{®} 80, 1 g; cetyl alcohol, 24 g; fragrance, 2 ml or QS
C. 7% w/w DPP spray formulation: almond oil, 100 g; propyl myristate, 100 g; DPP, 15 g; colloidal silica, 3 g; fragrance, 2 g or QS

Each of the formulations is tested for long term stability at room temperature (*e.g*., about 20° C) in transparent glass containers. Parameters assessed include: (1) physical stability (*e.g*., color change, consistency); (2) chemical stability (*e.g*., auto-oxidation, chemical changes in oil component(s)); (3) antimicrobial activity (*e.g*., microbiological (*e.g*., bacterial, fungal) counts.

### Example 2

DPP powder was subjected to Gamma Radiation at 25 kilogray (kGy) to eliminate any contamination that could occur during the process of collection, purification, handling, shipping, etc. The material was tested for microbiological quality according to USP 61 and 62. Total counts of aerobic bacteria, yeast, and mold were less than 10 CFU/g or ml, and *Escherichia coli, Pseudomonas aeruginosa, Staphylococcus aureus, Salmonella sp.,* and Bile tolerant Gram negative bacteria were absent.

The results indicated that the material meets the USP requirement criteria for microbiological quality of non-sterile cutaneous preparations.

### Example 3

A microbiological Challenge Test was carried out on the irradiated DPP described in Example 2.

The Challenge Test is a method to evaluate the effectiveness of a preserving system used in the formulation of a non-sterile cosmetic, drug, detergent, or similar product. By introducing artificial contamination, the environmental microbial pollution that products undergo during manufacturing, storage, and consumer use is reproduced. This provides information regarding the products resistance to microbial attacks and on its stability.

In the assay described in this example, the conditions surpassed concentrations of microorganisms that are typically found in the environment. The products were contaminated with various microbial strains, and their reduction in growth was evaluated at different time points.

The inoculum included microbial strains at concentrations shown in Table 1.

**Table 1**

| **Strain** | **Growth Medium** | **Inocula Concentration (CFU/g)** |
|---|---|---|
| *Escherichia coli* ATCC 8739 | Casein soya bean digest agar | 5.9 × 10⁵ |
| *Pseudomonas aeruginosa* ATCC 9027 | Casein soya bean digest agar | 1.5 × 10⁵ |
| *Staphylococcus aureus* ATCC 6538 | Casein soya bean digest agar | 9.6 × 10⁵ |
| *Candida albicans* ATCC 10231 | Sabouraud-dextrose agar | 4.4 × 10⁵ |
| *Aspergillus brasiliensis* ATCC 16404 | Sabouraud-dextrose agar | 5.9 × 10⁵ |

The different microbial strains were suspended in a physiologic solution and inoculated in the tested product at a final concentration of 10⁵ - 10⁶ for all strains. The treated samples were then stored at room temperature protected from light until plated for microbial count. The microbial count at different end points was carried out by diluting 1 g/ml of product up to 1 × 10⁶ times and plating each dilution in a petri dish with selective agar medium. To evaluate microbial reduction over time, plate counts were carried out at three different end times, 7, 14, and 28 days after inoculation. The results are shown in Table 2.

**Table 2**

| **Strain** | ***E. coli*** | ***P. aeruginosa*** | ***S**. **aureus*** | ***C. albicans*** | ***A. brasiliensis*** |
|---|---|---|---|---|---|
| **CFU Inoculum** | **5.9 x 10⁵** | **1.5 x 10⁵** | **9.6 x 10⁵** | **4.4 x 10⁵** | **1.9** x **10⁵** |
| CFU 7 days | 1.8 x 10⁴ | 1.0 x 10³ | 4.0 x 10³ | <10 | 9.0 x 10³ |
| Microbial reduction (log) | 1.52 | 2.18 | 2.38 | >4.64 | 1.32 |
| Reduction effectiveness (%) | 96.95 | 99.33 | 99.58 | >99.99 | 95.25 |
| CFU 14 days | 3.3 x 10⁴ | 1.0 x 10³ | 2.1 x 10⁴ | <10 | 2.0 x 10³ |
| Microbial reduction (log) | 1.25 | 2.18 | 1.66 | >4.64 | 1.98 |
| Reduction effectiveness (%) | 94.410 | 99.330 | 97.81 | >99.99 | 98.95 |
| CFU 28 days | 1.4 x 10⁴ | <10 | 4.0 x 10³ | <10 | 2.1 x 10⁴ |
| Microbial reduction (log) | 1.62 | >4.18 | 2.38 | 4.64 | 0.96 |
| Reduction effectiveness (%) | 97.63 | >99.99 | 99.58 | >99.99 | 88.95 |

The results of this analysis indicated that DPP satisfied the requirements of the preservation of efficacy test for oral products according to USP regulations.

### Example 4

Three anhydrous formulations of DPP were made for a serum, a cream, and a balm. Tables 3-5 below provide the detailed ingredients and concentrations used.

**Table 3. DPP ANHYDROUS CREAM (OINTMENT)**

| **Ingredient** | **Concentration (%)** |
|---|---|
| BBS-C All purpose vegetable oil | 35.0500 |
| LANOLIN | 6.7000 |
| TOCOPHEROL ACETATE | 0.2500 |
| COCO GLYCERIDES | 5.0000 |
| BEES WAX | 1.5000 |
| AVOCADO OIL | 23.0000 |
| DPP_{X} (Date Palm Pollen) | 20.0000 |
| HYDROGENATED VEGTABLE GLYCERIDES | 7.5000 |
| LAVENDER WATER LILY T-5567 | 1.0000 |

**Table 4. DPP ANHYDROUS BALM**

| **Ingredient** | **Concentration (%)** |
|---|---|
| BEES WAX | 10.5000 |
| AVOCADO OIL | 47.2500 |
| HYDROGENATED VEGTABLE GLYCERIDES | 8.0000 |
| ROSEMARY OIL | 5.0000 |
| TOCOPHEROL ACETATE | 0.2500 |
| COCO GLYCERIDES | 10.0000 |
| JOJOBA OIL | 5.0000 |
| DPP_{X} (Date Palm Pollen) | 10.0000 |
| CANDELILLA WAX | 3.0000 |
| LAVENDER WATER LILY T-5567 | 1.0000 |

**Table 5. DPP ANHYDROUS SERUM**

| **Ingredient** | **Concentration (%)** |
|---|---|
| AVOCADO OIL | 68.7500 |
| JOJOBA OIL | 5.0000 |
| ROSEMARY OIL | 5.0000 |
| ALOE OIL | 2.0000 |
| SESAME OIL | 2.0000 |
| TOCOPHEROL ACETATE | 0.2500 |
| DPPx (Date Palm Pollen) | 10.0000 |
| HYDROGENATED VEGTABLE GLYCERIDES | 6.0000 |
| LAVENDER WATER LILY T-5567 | 1.0000 |

Accelerated stability testing was carried on these formulations to determine any change in the physicochemical properties. These properties include the appearance, color, odor, UV effect, pH, specific gravity, and application on the skin at 40°C.

Results for 4 weeks of testing are shown below in Tables 6-8. The results indicate that there was no change in these properties.

**Table 6. DPP Anhydrous Cream**

| **TEST** | **Test Method** | **SPECIFICATION** | **One WEEK Sept 13, 2022** | **Two Weeks Sept 20, 2022** | **Three WEEKS Sept 27, 2022** | **Four WEEKS Oct 04 2022** |
|---|---|---|---|---|---|---|
| **Appearance** | TM-009.a | Soft Solid | Soft Solid | Soft Solid | Soft Solid | Soft Solid |
| **Color** | TM-010.a | Yellowish/ Greenish | EQ to Standard | EQ to Standard | EQ to Standard | EQ to Standard |
| **Odor** | TM-005.b | Characteristic | Characteristic | Characteristic | Characteristic | Characteristic |
| **UV Light** | SOP 147.b | No color change | No color change | No color change | No color change | No color change |
| **pH at 40°C** | TM-001.a | 6.20- 7.5 | 6.75 | 6.81 | 6.88 | 6.91 |
| **Viscosity @ 40 °C (LVT, Spd #3, at 12 rpm)** | TM-003.a | 8000 - 10000 CPs | 8600 CPs | 8500 CPs | 8.600 CP | 8,700 CPs |
| **Specific Gravity at 25 °C** | TM-014.a | 1.1000-1.2000 | 1.1420 | 1.1470 | 1.1490 | 1.1940 |
| **% Solid** | TM- 019 | 99.0 % Min | 99.83% | 99.80% | 99.77% | 99.53% |
| **Application on hand** | Smooth | Very smooth | Very smooth | Very smooth | Very smooth | Very smooth |

| | | | | | | |
|---|---|---|---|---|---|---|
| **The product was placed in an oven at 40°C with RH of 75% for the duration of testing.** | | | | | | |

**Table 7. DPP Anhydrous Balm**

| **TEST** | **Test Method** | **SPECIFICATION** | **One WEEK Sept 13, 2022** | **Two WEEKS Sept 20, 2022** | **Three WEEKS Sept 27, 2022** | **Four WEEKS Oct 04 2022** |
|---|---|---|---|---|---|---|
| **Appearance** | TM-009.a | Solid | Solid | Solid | Solid | Solid |
| **Color** | TM-010.a | Yellowish/ | EQ to Standard | EQ to Standard | EQ to Standard | EQ to Standard |
| | | Greenish | | | | |
| **Odor** | TM-005.b | Characteristic | Characteristic | Characteristic | Characteristic | Characteristic |
| **UV Light** | SOP 147.b | No color change | No color change | No color change | No color change | No color change |
| **pH at 40 °C** | TM-001.a | 6.20- 7.5 | 7.22 | 7.18 | 7.25 | 7.30 |
| **Viscosity @ 40 °C (LVT, Spd #3, at 12 rpm)** | TM-003.a | 10,000 CPs Min | 12000 CPs | 11700 CPS | 11,200 CPs | 11,300 CPs |
| **Specific Gravity at 25 °C** | TM-014.a | 1.1000-1.2000 | 1.1420 | 1.1470 | 1.1490 | 1.1640 |
| **% Solid** | TM- 019 | 99.0 % Min | 99.91% | 99.93% | 99.87% | 99.63% |
| **Application on Lips** | **Smooth** | smooth | smooth | smooth | smooth | smooth |

| | | | | | | |
|---|---|---|---|---|---|---|
| **The product was placed in an oven at 40°C with RH of 75% for the duration of testing.** | | | | | | |

**Table 8. Anhydrous Serum**

| **TEST** | **Test Method** | **SPECIFICATION** | **One WEEK Sept 13, 2022** | **Two WEEKS Sept 20, 2022** | **Three WEEKS Sept 27,2022** | **Four WEEKS Oct 04 2022** |
|---|---|---|---|---|---|---|
| **Appearance** | TM-009.a | Semi Solid | Soft semi solid | Soft semi solid | Soft semi solid | Soft semi solid |
| **Color** | TM-010.a | Yellowish/ Greenish | EQ to Standard | EQ to Standard | EQ to Standard | EQ to Standard |
| **Odor** | TM-005.b | Characteristic | Characteristic | Characteristic | Characteristic | Characteristic |
| **UV Light** | SOP 147.b | No color change | EQ to Standard | EQ to Standard | EQ to Standard | EQ to Standard |
| **pH at 40°C** | TM-001.a | 6.20- 7.5 | 7.20 | 7.10 | 7.20 | 7.00 |
| **Viscosity @ 40 °C (LVT, Spd #3, at 12 rpm)** | TM-003.a | 4000-6000 CPs | 4200 CPs | 4300 CPs | 4400 CPS | 4400 CPS |
| **Specific Gravity at 25 °C** | TM-014.a | 1.1000-1.2000 | 1.1200 | 1.1230 | 1.1250 | 1.1240 |
| % **Solid** | TM- 019 | 99.0 % Min | 99.70% | 99.45% | 99.51% | 99.39% |
| **Application on face** | **Smooth** | Very smooth | Very smooth | Very smooth | Very smooth | Very smooth |

| | | | | | | |
|---|---|---|---|---|---|---|
| **The product was placed in an oven at 40°C with RH of 75% for the duration testing.** | | | | | | |

Although the foregoing invention has been described in some detail by way of illustration and examples for purposes of clarity of understanding, it will be apparent to those skilled in the art that certain changes and modifications may be practiced without departing from the spirit and scope of the invention. Therefore, the description should not be construed as limiting the scope of the invention.

All publications, patents, and patent applications cited herein are hereby incorporated by reference in their entireties for all purposes and to the same extent as if each individual publication, patent, or patent application were specifically and individually indicated to be so incorporated by reference.

## Claims

1. A composition comprising pollen from a palm species of the genus *Phoenix* or an extract thereof,
wherein said composition is resistant to microbial growth and/or oxidative damage or color change over a period of time of at least about 3 years at about 20 °C,
wherein said microbial growth and/or oxidative damage is prevented or is reduced in comparison to an identical composition that does not comprise said pollen or extract thereof, and
wherein said pollen or extract thereof acts as a stabilizer and/or as a preservative in said composition,
optionally wherein said pollen or extract thereof is from *Phoenix dactylifera* L, optionally wherein the composition is anhydrous, optionally wherein the composition comprises a lipid.

2. The composition of claim 1, wherein the pollen or extract thereof is included in the composition at a concentration of about 2% (w/w) to about 20% (w/w).

3. The composition of claim 1 or 2, wherein said composition does not substantially degrade over said period of time, as indicated by a reduction or elimination of changes in consistency, color, and/or smell, in comparison to an identical composition that does not comprise said pollen or extract thereof.

4. The composition of any of claims 1 to 3,
wherein said composition is a drug or cosmetic composition, and wherein said microbial growth comprises bacterial growth that is limited to a maximum of 100 CFU per g or ml and/or fungal growth that is limited to a maximum of 10 CFU per g or ml over said period of time, or
wherein said composition is a food composition, and wherein said microbial growth comprises bacterial and/or fungal growth that is limited to a maximum of 10⁶ CFU per g or ml.

5. The composition of any of claims 1 to 4, wherein the composition is stored in a transparent or translucent container for said period of time and is not shielded from exposure to light.

6. The composition of any of claim 1 to 5, wherein the composition is an anhydrous liquid, suspension, emulsion, or powder composition, optionally wherein the composition is formulated as a skin care, cosmetic, food, or pharmaceutical composition.

7. The composition of any of claims 1 to 6, comprising one or more constituent to facilitate formulation, stability, and/or topical application of the composition, optionally wherein the one or more constituent comprises a flow regulating agent, a filler, an excipient, an alcohol, a suspending agent, an oil phase, a humectant or a thickener.

8. The composition of any of claims 1 to 7, comprising one or more component to facilitate topical application of the composition, wherein the composition is in the form of a powder, a compact disc, a suspension, a cream, a lotion, an ointment, a gel, a foam, a paste, an aerosol, a body wash, a hair product, a sunscreen, a lipstick, an emulsion, a cosmetic, or an anhydrous absorption base composition,
optionally wherein the composition comprises one or more constituent selected from colloidal silica, titanium dioxide, isopropyl alcohol, benzalkonium chloride, stearic acid, cetyl alcohol, isopropyl palmitate, methylparaben, propylparaben monostearate, sorbitol, polysorbate, milk, coconut oil, almond oil, lanolin, lecithin, and beeswax, optionally wherein the composition further comprises a fragrance.

9. The composition of any of claims 1 to 7, comprising one or more component to facilitate oral administration of the composition, wherein the composition is in the form of a powder, a tablet, a capsule, an aerosol suspension, or an effervescent powder or tablet.

10. The composition of any of claims 1 to 7, formulated as a food composition, wherein the composition is in the form of an oil, a dehydrated food, or a powdered food or baking mix.

11. A method of preserving and/or stabilizing a composition, said method comprising adding pollen or an extract thereof from a palm species of the genus *Phoenix* to said composition, wherein said preserving and/or stabilizing comprises prevention or reduction of microbial growth and/or oxidative damage or color change over a period of time of at least about 3 years at about 20 °C, in comparison to an identical composition that does not comprise said pollen or extract thereof,
optionally wherein said pollen or extract thereof is from *Phoenix dactylifera* L., optionally wherein the composition is anhydrous.

12. The method of claim 11, wherein the pollen or extract thereof is added to the composition at a concentration of about 2% (w/w) to about 10% (w/w).

13. The method of claim 11, wherein said composition is stored in a transparent or translucent container for said period of time and is not shielded from exposure to light.

14. A preservative composition comprising pollen from a palm species of the genus *Phoenix* or an extract thereof and at least one diluent, wherein said composition reduces or eliminates microbial growth and/or oxidative damage in a composition or product into which the preservative composition is incorporated, in comparison to an identical composition or product that does not comprise the pollen or extract thereof.

15. The preservative composition according to claim 24,
wherein when said preservative composition is incorporated into a drug or cosmetic composition, microbial growth comprises bacterial growth that is limited to a maximum of 100 CFU per g or ml and/or fungal growth that is limited to a maximum of 10 CFU per g or ml over a period of time of at least about 3 years at about 20 °C, or
wherein when said preservative composition is incorporated into a food composition, microbial growth comprises bacterial and/or fungal growth that is limited to a maximum of 10⁶ CFU per g or ml over a period of time of at least about 3 years at about 20 °C,
optionally wherein said preservative composition is incorporated into said drug, cosmetic, or food product at a concentration of about 2% (w/w) to about 20% (w/w).
